(19) Europäisches Patentamt
European Patent Office
Office européen des brevets

(11) EP 3 326 636 A1

(12) EUROPEAN PATENT APPLICATION
published in accordance with Art. 153(4) EPC

(43) Date of publication:
30.05.2018 Bulletin 2018/22

(21) Application number: 16827722.6

(22) Date of filing: 14.07.2016

(51) Int Cl.:
A61K 35/62 (2006.01)
A23L 33/10 (2016.01)
A61P 3/00 (2006.01)
A61P 25/28 (2006.01)

(86) International application number:
PCT/JP2016/070902

(87) International publication number:
WO 2017/014162 (26.01.2017 Gazette 2017/04)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 21.07.2015 JP 2015144319

(71) Applicant: Well Stone Co.
Miyazaki-shi, Miyazaki 889-1701 (JP)

(72) Inventors:
- ISHII Yoichi
Miyazaki-shi
Miyazaki 880-0014 (JP)
- OKAMOTO Takeshi
Miyazaki-shi
Miyazaki 880-0908 (JP)
- ISHII Sayaka
Miyazaki-shi
Miyazaki 880-0014 (JP)

(74) Representative: Boult Wade Tennant
Verulam Gardens
70 Gray's Inn Road
London WC1X 8BT (GB)

# (54) AMYLOID ß FIBRIL DECOMPOSING AGENT, AND THERAPEUTIC OR PREVENTIVE AGENT AND THERAPEUTIC OR PREVENTIVE FOOD COMPOSITION FOR DISEASES CAUSED BY AMYLOID ß FIBRIL FORMATION

(57) An object of the present invention is to provide: an amyloid β fibril decomposing agent containing a natural product as an active ingredient; a therapeutic or preventive agent for a disease caused by amyloid β fibril formation; and a therapeutic or preventive food composition for a disease caused by amyloid β fibril formation. Provided are: an amyloid β fibril decomposing agent containing a dry powder, ground product and/or extract of an earthworm as an active ingredient; a therapeutic or preventive agent for a disease caused by amyloid β fibril formation; and a therapeutic or preventive food composition for a disease caused by amyloid β fibril formation. The disease caused by amyloid β fibril formation is preferably Alzheimer's disease.

FIG. 1

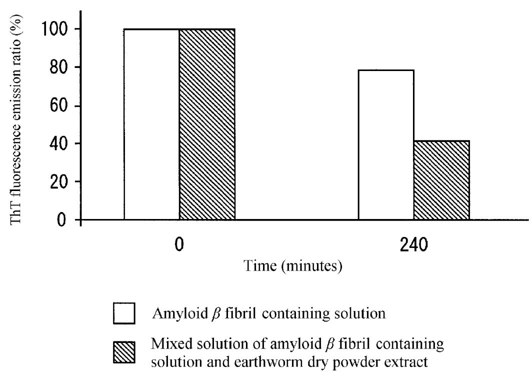


EP 3 326 636 A1

## Description

TECHNICAL FIELD

The present invention relates to: an amyloid β fibril decomposing agent; a therapeutic or preventive agent for a disease caused by amyloid β fibril formation; and a food composition for the treatment or prevention of a disease caused by amyloid β fibril formation.

BACKGROUND ART

Amyloid β (also referred as "amyloid β protein" or "amyloid β peptide) is a protein consisting of about 40 amino acid residues, and it is known that amyloid β fibrils, which are generated by aggregation and fibril formation of amyloid β, are accumulated in the brains of Alzheimer's disease patients. Amyloid β is a water-soluble protein that is discharged to the outside of cells and shows no toxicity to neurons by itself. However, since amyloid β becomes toxic to neurons only after the formation of amyloid β fibrils (see, for example, Patent Document 1), accumulation of amyloid β fibrils is believed to be one of the factors that cause Alzheimer's disease.
As main molecular species of amyloid β, amyloid β40 consisting of 40 residues whose C-terminal is Val and amyloid β42 consisting of 42 residues whose C-terminal is Ala are known and in normal neurons, amyloid β40 is produced nearly 10 times as much as amyloid β42 (see Non-patent Document 1). Amyloid β42 is highly agglutinative and known to dominantly accumulate also in the brains of Alzheimer's disease patients from the early stage. Further, as gene mutations associated with familial Alzheimer's disease, mutations existing in the amyloid β precursor protein (APP) such as the Iranian mutation (T714A) are known to increase the production ratio of highly agglutinative amyloid β42 without largely affecting the total production of amyloid β. Moreover, as such gene mutations, a mutation that causes an increase in the total production of amyloid β and a mutation that causes an increase in the agglutinability of amyloid β are also known.
So far, therapeutic agents for Alzheimer's disease which target amyloid β have been developed. For example, Patent Document 2 discloses an amyloid β protein production and secretion inhibitor comprising vinpocetine and a derivative thereof. In addition, Patent Document 3 discloses a peptide capable of inhibiting fibril formation of an amyloid β peptide.
Meanwhile, mainly in the Oriental countries, earthworm extracts and dry earthworm powders have been used since ancient times as preventive agents and therapeutic agents for various diseases, and examples of their use that have been known include bladder stone-reducing agents, bladder stone excretion-promoting agents, therapeutic agents for jaundice, oxytocics, tonic agents, hair growth agents, aphrodisiacs, antipyretics, therapeutic agents for convulsion, blood circulation promoters, therapeutic agents for hemiplegia, indirect analgesics, diuretics, antiasthmatics and antihypertensive agents.
However, no report has been made on the use of earthworms for the prevention and treatment of Alzheimer's disease.

RELATED ART DOCUMENTS

PATENT DOCUMENTS

Patent Document 1: Japanese Patent No. 4227275
Patent Document 2: Japanese Unexamined Patent Application Publication No. H8-283157
Patent Document 3: Japanese Patent No. 5464383

NON-PATENT DOCUMENTS

Non-patent Document 1: Asami-Odaka, A et al., Biochemistry: 1995, 34(32); 10272-10278
Non-patent Document 2: Iwatsubo, T et al., Neuron (1994), 13(1); 45-53)

## SUMMARY OF THE INVENTION

### PROBLEMS TO BE SOLVED BY THE INVENTION

The prevention and treatment of Alzheimer's disease is believed to involve drug administration over a long time; therefore, a drug that is safe and has little side effects is particularly necessary, and there is thus a demand for a naturally derived preventive or therapeutic agent which can inhibit amyloid β fibril formation.

In view of the above, an object of the present invention is to provide: an amyloid β fibril decomposing agent comprising a natural product as an active ingredient; a preventive or therapeutic agent for diseases caused by amyloid β fibril formation; and a food composition for the treatment or prevention of diseases caused by amyloid β fibril formation.

### MEANS FOR SOLVING THE PROBLEMS

That is, the amyloid β fibril decomposing agent according to the present invention is characterized by comprising a dry powder, ground product and/or extract of an earthworm as an active ingredient.

The therapeutic or preventive agent for a disease caused by amyloid β fibril formation according to the present invention is characterized by comprising the above-described amyloid β fibril decomposing agent.

The therapeutic or preventive food composition for a disease caused by amyloid β fibril formation according to the present invention is characterized by comprising the above-described amyloid β fibril decomposing agent.

In the therapeutic or preventive agent for a disease caused by amyloid β fibril formation according to the present invention, it is preferred that the disease caused by amyloid β fibril formation be Alzheimer's disease.

In the therapeutic or preventive food composition for a disease caused by amyloid β fibril formation according to the present invention, it is preferred that the disease caused by amyloid β fibril formation be Alzheimer's disease.

The method of producing an amyloid β fibril decomposing agent according to the present invention is characterized by comprising the use of a dry powder, ground product and/or extract of an earthworm.

It is preferred that the method of producing an amyloid β fibril decomposing agent according to the present invention comprise the steps of: bringing a live earthworm into contact with a chloride of at least one metal selected from the group consisting of potassium, sodium, magnesium and calcium; and subsequently bringing the live earthworm into contact with a powder-form hydroxycarboxylic acid, diluting the resultant with water to adjust the pH to 2 to 5, maintaining the resulting dilution for 3 to 180 minutes, washing the live earthworm with water, grinding the live earthworm, freeze-drying the resulting ground product, dissolving the thus freeze-dried product in water or an aqueous ethanol solution, and then removing or separating insoluble fractions.

It is also preferred that the method of producing an amyloid β fibril decomposing agent according to the present invention comprise the steps of: bringing a live earthworm into contact with a chloride of at least one metal selected from the group consisting of potassium, sodium, magnesium and calcium; and subsequently immersing and maintaining the live earthworm for 3 to 180 minutes in an aqueous hydroxycarboxylic acid solution having an adjusted pH of 2 to 5, washing the live earthworm with water, grinding the live earthworm, freeze-drying the resulting ground product, dissolving the thus freeze-dried product in water or an aqueous ethanol solution, and then removing or separating insoluble fractions.

The method of producing a therapeutic or preventive agent for a disease caused by amyloid β fibril formation according to the present invention is characterized by comprising the use of a dry powder, ground product and/or extract of an earthworm.

The method of producing a therapeutic or preventive food composition for a disease caused by amyloid β fibril formation according to the present invention is characterized by comprising the use of a dry powder, ground product and/or extract of an earthworm.

The method of decomposing amyloid β fibrils according to the present invention is characterized by comprising the use of a dry powder, ground product and/or extract of an earthworm.

The dry powder, ground product or extract of an earthworm according to the present invention is for the use in the treatment of a disease caused by amyloid β fibril formation.

The method of treating or preventing a disease caused by amyloid β fibril formation according to the present invention is characterized by comprising administration of a dry powder, ground product and/or extract of an earthworm to a subject in an effective dose.

### EFFECTS OF THE INVENTION

According to the present invention, an amyloid β fibril decomposing agent comprising a natural product as an active ingredient, a preventive or therapeutic agent for diseases caused by amyloid β fibril formation, and a food composition for the treatment or prevention of diseases caused by amyloid β fibril formation can be provided.

BRIEF DESCRIPTION OF THE DRAWINGS

FIG. 1 is a graph showing the comparison of the amount of fluorescence emitted by thioflavin T (hereinafter, abbreviated as "ThT") between an amyloid β fibril containing solution and a mixed solution of the amyloid β fibril containing solution and a dry earthworm powder extract.

MODE FOR CARRYING OUT THE INVENTION

In the method of the present invention, the earthworm used as a raw material is not particularly restricted, and examples of earthworms that can be used include *Lumbricus rubellus, Lumbricus terrestris, Eisenia foetida, Allolobophora caliginosa, Dendrobaena octaedra, Allolobophora japonica* Michaelsen, *Drawida hattamimizu* Hatai, *Pheretima divergens* Michaelsen, *Pheretima communissima, Pheretima agrestis, Pheretima sieboldi* Horst, *Pheretima hilgendorfi, Pontodrilus matsushimensis* Iizuka, *Tubifex hattai* Nomura, and *Limnodrilus gotoi* Hatai (= *L. Socialis* Stephenson).

In the present invention, the term "dry powder" of an earthworm means powder obtained by drying a ground product or extract of an untreated or pretreated earthworm. The term "ground product" of an earthworm means an untreated or pretreated earthworm ground into a liquid or paste form. The term "extract" of an earthworm means an extract obtained by dissolving an untreated or pretreated earthworm or a ground product thereof in water or an organic solvent and subsequently removing or separating insoluble fractions. The pretreatment is not particularly restricted, and examples thereof include the below-described treatment for removal of dirt and the like. Further, the dry powder, ground product and extract of an earthworm may also be subjected to a post-treatment, examples of which include granulation, filtration, purification, concentration, dilution and pH adjustment.

The grinding method for obtaining a ground product of an earthworm is not particularly restricted, and grinding can be performed by using, for example, a homogenizer, a blender, a homomixer, a grinder or a high-pressure cell crushing apparatus.

The extraction method for obtaining an extract of an earthworm is not particularly restricted, and extraction can be performed by, for example, dissolving a dry powder or ground product of the earthworm in an extraction solvent and subsequently removing or separating insoluble fractions. Examples of the extraction solvent include water, aqueous solutions, and organic solvents such as ethanol, acetone and ethyl acetate, and these extraction solvents may be used individually, or two or more thereof may be used in combination. Thereamong, water, ethanol or an aqueous ethanol solution is preferably used.

The drying method for obtaining a dried product of an earthworm is not particularly restricted, and drying can be performed by a drying method such as freeze-drying, heat-drying or spray-drying. Thereamong, freeze-drying is preferred for the below-described reasons.

In the present invention, the dry powder, ground product or extract of the earthworm can be incorporated in an effective amount in accordance with the purpose thereof. The appropriate amount depends on a variety of factors such as the intended purpose, the route and mode of administration and the production method of the dry powder or the like of the earthworm; however, for the purpose of preventing a disease caused by amyloid β fibril formation or treating a mild disease, the appropriate amount is preferably 1 to 15,000 mg/day, more preferably 12 to 1,800 mg/day, still more preferably 120 to 180 mg/day, in terms of the weight of the dry powder of the earthworm obtained by removing the digested matters remaining in the digestive tract of the earthworm as well as the dirt and the like adhering to the skin of the earthworm as described below, grinding the earthworm and then freeze-drying the resulting ground product. Further, for the purpose of treating a severe disease caused by amyloid β fibril formation, the appropriate amount is preferably 1 to 15,000 mg/day, more preferably 18 to 3,600 mg/day, still more preferably 180 to 360 mg/day.

The forms of the amyloid β fibril decomposing agent, therapeutic agent, preventive agent and food composition of the present invention are not particularly restricted and can be any of a solid form, a powder form, a semisolid form and a liquid form.

In the present invention, the dry powder, ground product or extract of the earthworm can be used as is. Alternatively, particularly the amyloid β fibril decomposing agent, therapeutic agent and preventive agent of the present invention may contain a pharmaceutically acceptable carrier and can be administered orally or parenterally (e.g., intravenous administration or direct administration to the affected site) in the form of a tablet, a granule, a powder, a capsule, a soft capsule, a liquid, an injectable, a suppository, a sustained release agent or the like. As the pharmaceutically acceptable carrier, for example, an excipient, a binding agent, a disintegrant, a fluidizing agent, a lubricant, a coating agent, a suspending agent, a colorant, a sweetening agent or a surfactant can be used, and the resultant can be made into the form of an ordinary pharmaceutical preparation in accordance with a known method. Further, other therapeutic and preventive component(s) and pharmaceutically acceptable additive(s) may also be incorporated.

In the present invention, particularly in the amyloid β fibril decomposing agent and food composition of the present invention, an additive(s) usually used in food products may also be incorporated. Examples of additives that can be used include an excipient, a binding agent, a disintegrant, a fluidizing agent, a lubricant, a coating agent, a suspending agent, a colorant, a sweetening agent and a surfactant, and the resultant can be made into the form of an ordinary food composition in accordance with a known method. Further, other food product(s) or food-derived component(s) may be incorporated as well.

In the present invention, among dry powders, ground products and extracts of earthworms, from the standpoint of the storage stability in the production process, it is preferred to use a dry powder of an earthworm. The dry powder of an earthworm may be dissolved and/or dispersed in a liquid such as water in advance, and the resultant may be subsequently mixed with other component(s), examples of which include conventional carriers and additives that are used pharmaceutically and/or in food products.

In the present invention, the disease caused by amyloid $\beta$ fibril formation is not particularly restricted, and examples thereof include Down syndrome in addition to Alzheimer's disease.

For oral administration of an earthworm as a raw material, it is preferred to remove the digested matters remaining in the digestive tract of the earthworm, the dirt adhering to the skin and the like. In the present invention, the method for such removal is not particularly restricted, and the removal can be performed by a known method. For example, a method of allowing a live earthworm to excrete yellow soil contained in the digestive tract by immersing the earthworm into an aqueous solution of an alkali salt such as a sodium salt or a potassium salt (method described in Japanese Unexamined Patent Application Publication Nos. H1-47718, H1-47719, H1-47720 and H1-268639) or a method of removing castings from the digestive tract of a live earthworm by leaving the earthworm in an aqueous acid solution maintained at 6 to 26°C for 0.1 to 5 hours (method described in Japanese Unexamined Patent Application Publication No. H3-72427) can be employed.

In the present invention, as a removal method, it is preferred to bring the earthworm into contact with the below-described metal chloride and/or hydroxycarboxylic acid.

The metal chloride is a chloride of at least one metal selected from the group consisting of potassium, sodium, magnesium and calcium. That is, the metal chloride is at least one selected from the group consisting of potassium chloride, sodium chloride, magnesium chloride and calcium chloride. Further, the metal chloride may also be a mixture of these metal chlorides, or a mixture of one or more of these metal chlorides and other harmless component(s) that can be added to food products. Examples of such mixtures include dietary salts, rock salts and bay salts. The metal chloride can be used by sprinkling it in a powder form over a live earthworm, and this causes a contact between the earthworm and the metal chloride.

After allowing the metal chloride to come into contact with the live earthworm, it is preferred to bring the live earthworm into contact with a hydroxycarboxylic acid in the below-described manner. Alternatively, the earthworm may be brought into contact with a hydroxycarboxylic acid in the below-described manner without a preceding contact with the metal chloride.

The contact with the hydroxycarboxylic acid can also be made by sprinkling the hydroxycarboxylic acid in a powder form over the live earthworm. Alternatively, the live earthworm may be immersed in an aqueous solution of the hydroxycarboxylic acid that has a pH of 2 to 5. In cases where the contact with the hydroxycarboxylic acid is made after a contact with the metal chloride, it is preferred that the contact with the hydroxycarboxylic acid be made promptly after the contact with the metal chloride. It is also preferred that the earthworm be washed with water before being brought into contact with the hydroxycarboxylic acid. By removing the metal chloride by washing with water and then bringing the earthworm into contact with the hydroxycarboxylic acid, a dry earthworm powder having a high enzyme activity can be obtained. When the earthworm is washed with water before the contact with the hydroxycarboxylic acid, the washing of the earthworm with water is performed preferably within 30 minutes, more preferably within 20 minutes, after the initiation of the contact with the metal chloride. The method of washing the earthworm with water is not particularly restricted, and a known method can be employed.

If live earthworms are left in contact with hydroxycarboxylic acid powder for a long time, the earthworms are killed, so that their vital functions are lost and the digested matters in their digestive tracts are no longer excreted. Therefore, it is preferred to dilute the hydroxycarboxylic acid with water as soon as possible, preferably within 30 seconds, more preferably within 20 seconds, so as to adjust the pH to a range of 2 to 5.

Since a hydroxycarboxylic acid creates a living environment unpleasant to earthworms, live earthworms, following their self-preservation instinct, try to improve the living environment through discharge of body fluids and excretion. Further, since hydroxycarboxylic acids have disinfecting properties, they are expected not only to play a role in promoting excretion of digested matters and the like remaining in the digestive tract as described above, but also to have an effect of killing bacteria adhering to earthworms.

In the above-described method, any crystalline hydroxycarboxylic acid can be used regardless of the number of its hydroxy groups and carboxyl groups, as long as it assumes a crystalline form under the conditions of its use. That is, the crystalline hydroxycarboxylic acid may be any of monohydroxy monocarboxylic acids, monohydroxy polycarboxylic acids, polyhydroxy monocarboxylic acids and polyhydroxy polycarboxylic acids.

Examples of the hydroxycarboxylic acid used in the present invention include glycolic acid, lactic acid, acetic acid, $\beta$-hydroxypropionic acid, $\alpha$-hydroxy-*n*-butyric acid, $\beta$-hydroxy-*n*-butyric acid, $\alpha$-hydroxy-*n*-valeric acid, $\beta$-hydroxy-

*n*-valeric acid, malic acid, α-methylmalic acid, α-hydroxyglutaric acid, *β*-hydroxyglutaric acid, citric acid, malonic acid, and succinic acid. Thereamong, lactic acid, acetic acid, malic acid, citric acid, malonic acid and succinic acid are preferred because they can be used in food products and easily obtained. The above-described hydroxycarboxylic acids may be used individually, or two or more thereof may be used in combination.

Water constitutes 65% of the tissues of a live earthworm. Although the self-preservation functions of a live earthworm remain effective for a certain time, the death of the live earthworm results in the onset of enzyme activities; therefore, it is required to carefully control the period of placing the live earthworm under an unpleasant living environment. The duration of this period varies depending on the conditions; however, it is usually in a range of 3 to 180 minutes.

It is preferred that the thus hydroxycarboxylic acid-treated live earthworm be washed with water and then ground into a liquid-form or paste-form ground product. The washing is preferably performed with pure water. The washing method is not particularly restricted, and a known washing method with water can be employed. The total time of the treatment process before the grinding, that is, the duration of the period from the sprinkling of the metal chloride on the live earthworm to the completion of the removal of the hydroxycarboxylic acid by washing with water, is preferably not longer than 240 minutes.

The grinding method is not particularly restricted and, for example, the grinding is usually performed at 1 to 25°C using a homogenizer, a blender, a homomixer, a grinder, a high-pressure cell-crushing apparatus or the like. From the standpoint of inhibiting degradation of the earthworm components, it is preferred that the grinding be performed at a low temperature, more preferably at a temperature of 2 to 15°C.

The ground product obtained by grinding the earthworm is placed on a stainless-steel tray or the like and subjected to freeze-drying. In this process, a decomposition gas may be generated because the enzymes contained in the living body of the earthworm are inactive in the living cells but act instantaneously on dead cells. In order to inhibit the generation of a decomposition gas, it is preferred that, before being freeze-dried, the ground product be momentarily cooled rapidly and frozen at -18°C to -35°C so as to suppress the enzyme actions.

In this manner, for the preparation of earthworm powder without impairing the inherent pharmacological actions of the earthworm, it is preferred that the ground earthworm be quickly frozen. On the other hand, an excessively rapid freezing is not preferred because when the ground earthworm is frozen in an excessively short period of time, the impurities existing together with the proteins that are major components of an earthworm paste can form spots of unfrozen parts and thus may not be separated. Therefore, the freezing is performed at a low temperature of -18°C to -35°C over a period of preferably 20 to 240 hours, more preferably 50 to 170 hours.

For the freeze-drying, it is important to select conditions that allow removal of water as well as impurities without leaving any impurity. Accordingly, it is preferred that the freeze-drying be controlled under a pressure of 50 Pa or less while increasing the temperature stepwise in a range of -60°C to +90°C over a period of 10 to 60 hours.

As a freeze-drying method, for example, as described above, after freezing the ground product at a temperature of -18°C to -35°C over a period of 20 to 240 hours, the resultant is vacuum freeze-dried over a period of 10 to 60 hours while increasing the temperature in several steps in a range of -60°C to +90°C and reducing the pressure in several steps in a range of 25 to 40 Pa, whereby a sterile pale yellow dry earthworm powder can be obtained.

Further, it is also preferred to incorporate the steps of dissolving the thus freeze-dried ground product in water or an aqueous ethanol solution, and removing or separating insoluble fractions. The step of removing or separating insoluble fractions can be performed in the same manner as described above and comprise precipitation carried out by leaving the resulting solution to stand, centrifugation, filtration and the like. The step of dissolving the freeze-dried ground product in water or an aqueous ethanol solution is preferably performed with stirring or shaking. The time required for dissolution of the freeze-dried ground product in water is preferably 1 to 120 minutes, more preferably 5 to 80 minutes. The ethanol concentration of the aqueous ethanol solution is not particularly restricted; however, it is preferably 10 to 70% (v/v), more preferably 30 to 60%.

As the amyloid *β* fibril decomposing agent, therapeutic agent, preventive agent and food composition of the present invention, a supernatant obtained from water or aqueous ethanol solution in which freeze-dried ground earthworm is dissolved as described above may be used as is in the state of an aqueous solution, or may be used in the form of a concentrate after evaporating water therefrom. The supernatant may also be dried to be used in a powder form, and the powder obtained by drying the supernatant may be dissolved in water for use. Further, powder obtained by freeze-drying an earthworm paste can be used as is, without being dissolved in water or an aqueous ethanol solution.

In the present invention, as a removal method, before the treatment of placing live earthworms under an unpleasant environment, that is, before bringing live earthworms into contact with a metal chloride or a hydroxycarboxylic acid as described above, it is preferred that the live earthworms be transferred to a flat box such as a bread box and left to stand for 10 to 50 hours in a bright place, followed by removal of dirt adhering to the earthworm skin. The duration of leaving the live earthworms in a bright place is more preferably 12 to 24 hours. In this process, it is preferred that the amount of the live earthworms contained in the flat box be such an amount that the earthworms are piled up to a thickness of 30 to 60 mm, preferably 40 to 50 mm. Care should be taken so that the flat box contains no foreign matter such as sand or mud and, since earthworms are nocturnal and thus become active in their living activities in dark place and this may lead to their physical exhaustion, it is preferred to employ a light culture method or the like during the night so as to keep the flat box under a bright condition. This treatment makes the live earthworms exhibit their self-protection instinct and try to maintain their living environment by excreting the digested matters remaining in their digestive tracts, covering their entire body with the excrements and thereby preventing evaporation of water. Thus, by repeatedly striping off this covering dirt, namely excrements, by an appropriate means, the digested matters in the digestive tracts of the earthworms and the dirt adhering to their skin can be eventually removed.

The dirt adhering to the earthworms' skin can be stripped off by, for example, covering the live earthworms with a nonwoven fabric and allowing the dirt to adsorb to the fabric. By performing this process of leaving the earthworms in a bright place and removing the dirt adhering to their skin in combination with the above-described process of bringing the earthworms into contact with the a metal chloride and/or a hydroxycarboxylic acid, further excretion and removal of toxic matters from the earthworms' bodies can be expected.

In the present invention, as a method for obtaining a dry earthworm powder, the following methods are preferred particularly from the standpoint of the storage stability of the resulting dry powder.

(A-1) A method of producing a dry earthworm powder, the method comprising the steps of:

bringing a live earthworm into contact with a chloride of at least one metal selected from the group consisting of potassium, sodium, magnesium and calcium; and
subsequently bringing the live earthworm into contact with a powder-form hydroxycarboxylic acid, diluting the resultant with water to adjust the pH to 2 to 5, maintaining the resulting dilution for 3 to 180 minutes, washing the live earthworm with water, grinding the live earthworm, and then freeze-drying the thus obtained ground product.

(A-2) A method of producing a dry earthworm powder, the method comprising the steps of:

bringing a live earthworm into contact with a chloride of at least one metal selected from the group consisting of potassium, sodium, magnesium and calcium; and
subsequently immersing and maintaining the live earthworm for 3 to 180 minutes in an aqueous hydroxycarboxylic acid solution having an adjusted pH of 2 to 5, washing the live earthworm with water, grinding the live earthworm, and then freeze-drying the thus obtained ground product.

(A-3) The method of producing a dry earthworm powder according to the above-described (A-1) or (A-2), which further comprises the steps of: dissolving the thus freeze-dried ground product into water or an aqueous ethanol solution; removing or separating insoluble fractions; and then further freeze-drying the resultant.

Further, after the freeze-drying of the ground product obtained by grinding the live earthworm, from the standpoint of sterilization of the resulting dried product, the dried product may be heat-treated as well. The temperature of the heat treatment is preferably 110°C or higher but lower than 130°C. When the heating temperature is lower than 110°C, the dried product may not be sterilized sufficiently, whereas when the heating temperature is 130°C or higher, the enzymes contained in the dried earthworm product are inactivated and their activities are thus reduced, which is not preferred. The heating temperature is more preferably 115 to 125°C. The heating method is not particularly restricted, and examples thereof include a method of blowing hot air; a method using a heating jacket; a method of heating the subject on a tray or the like using a heater; and a method using a thermostat incubator. The heating time is preferably 30 seconds to 130 minutes, more preferably 30 minutes to 90 minutes, still more preferably 60 minutes to 90 minutes. An excessively short heating time may result in insufficient sterilization while an excessively long heating time may cause the loss of enzyme activities, neither of which is preferred. When enzymes contained in a liquid are subjected to the above-described heat treatment, the activities of the enzymes are lost; therefore, the heating treatment is preferably performed on a dry earthworm powder.

In the present invention, as a method for obtaining a ground product of an earthworm, the following methods are preferred.

(B-1) A method of producing a ground product of an earthworm, the method comprising the steps of:

bringing a live earthworm into contact with a chloride of at least one metal selected from the group consisting of potassium, sodium, magnesium and calcium; and
subsequently bringing the live earthworm into contact with a powder-form hydroxycarboxylic acid, diluting the resultant with water to adjust the pH to 2 to 5, maintaining the resulting dilution for 3 to 180 minutes, washing the live earthworm with water, and then grinding the live earthworm.

(B-2) A method of producing a ground product of an earthworm, the method comprising the steps of:

bringing a live earthworm into contact with a chloride(s) of a metal(s) selected from the group consisting of potassium, sodium, magnesium and calcium; and

subsequently immersing and maintaining the live earthworm for 3 to 180 minutes in an aqueous hydroxycarboxylic acid solution having an adjusted pH of 2 to 5, washing the live earthworm with water, and then grinding the live earthworm.

In the present invention, as a method for obtaining an earthworm extract, the following methods are preferred.

(C-1) A method of producing an earthworm extract, the method comprising the steps of:

bringing a live earthworm into contact with a chloride of at least one metal selected from the group consisting of potassium, sodium, magnesium and calcium; and
subsequently bringing the live earthworm into contact with a powder-form hydroxycarboxylic acid, diluting the resultant with water to adjust the pH to 2 to 5, maintaining the resulting dilution for 3 to 180 minutes, washing the live earthworm with water, grinding the live earthworm, freeze-drying the resulting ground product, dissolving the thus freeze-dried product in water or an aqueous ethanol solution, and then removing or separating insoluble fractions.

(C-2) A method of producing an earthworm extract, the method comprising the steps of:

bringing a live earthworm into contact with a chloride(s) of a metal(s) selected from the group consisting of potassium, sodium, magnesium and calcium; and
subsequently immersing and maintaining the live earthworm for 3 to 180 minutes in an aqueous hydroxycarboxylic acid solution having an adjusted pH of 2 to 5, washing the live earthworm with water, grinding the live earthworm, freeze-drying the resulting ground product, dissolving the thus freeze-dried product in water or an aqueous ethanol solution, and then removing or separating insoluble fractions.

## EXAMPLES

The present invention will now be described in more detail by way of examples thereof. The present invention, however, is not restricted by the following examples by any means. It is noted here that, unless otherwise specified, "%" used below is all based on mass.

[Preparation of Dry Earthworm Powder]

After leaving 30 kg of live red earthworms (*Lumbricus rubellus*) to stand in a bright place for 24 hours and stripping off dirt adhering to their skin, the live red earthworms were spread at a thickness of about 5 cm on a flat dish, and 250 g of sodium chloride was uniformly sprinkled thereon. The earthworms were washed with water 20 minutes thereafter. Then, 15 seconds after sprinkling 250 g of citric acid on the earthworms in the same manner, 30 L of pure water was added thereto for dilution. In this process, the pH of the resulting solution was 2.25 immediately after the addition of water and 2.74 after the completion of the dilution. When sprinkled with the citric acid powder, the earthworms excreted yellow body fluid at once. After the dilution with water, the earthworms were maintained in this state for 20 minutes. Next, the live earthworms were taken out of the resulting dirty aqueous citric acid solution, washed with water and subsequently ground at 10°C using a homogenizer to prepare an earthworm paste. Then, after subjecting this earthworm paste to vacuum degassing so as to remove the gases contained therein, the earthworm paste was transferred to a stainless-steel tray where the earthworm paste was instantaneously and rapidly cooled to -35°C and maintained at this temperature for 50 hours to be slowly frozen. The thus frozen earthworm paste was maintained at -35°C under a pressure of 0 Pa for 2 hours. Thereafter, the earthworm paste was heated and dried at 25°C under a pressure of 40 Pa for 10 hours, at 40°C under a pressure of 35 Pa for 14 hours and then at 65°C under a pressure of 35 Pa for 12 hours and lastly, the resultant was maintained at a temperature of 80°C and a pressure of 25 Pa for 6 hours, thereby vacuum freeze-drying the earthworm paste. By this treatment, a pale-yellow dry earthworm powder having a water content of 8% by mass was obtained.

[Preparation of Dry Earthworm Powder Extract]

Next, 25 g of the thus obtained dry earthworm powder was collected and 500 mL of distilled water was added thereto, followed by 1-hour extraction with stirring at room temperature. The thus obtained extract was centrifuged (10,000×g, 4°C, 15 minutes), and the resulting supernatant was collected.

The thus obtained supernatant of the extract was diluted with a 50 mM phosphate buffer (pH 7.0) containing 150 mM of sodium chloride (hereinafter, referred to as "buffer") to a concentration of 25 mg/mL in terms of the amount of the dry earthworm powder. Then, the dilution product was centrifuged (10,000×g, 4°C, 15 minutes) and the resulting supernatant was collected, whereby a dry earthworm powder extract was prepared.

[Preparation of Amyloid β Fibrils]

Amyloid β42 (manufactured by Peptide Institute, Inc.; Amyloid β-Protein (Human, 1-42), 4349-v, Lot. 650312) was dissolved in 75% trifluoroethanol to a concentration of 0.5 mM, and the concentration was further adjusted to 25 μM with a 50 mM phosphate buffer (pH 7.0) containing 150 mM of potassium chloride. This solution was incubated at 25°C for 2 weeks to allow fibril formation to take place. The resultant was centrifuged (14,000×g, 25°C, 20 minutes) to recover the thus formed fibrils, and the fibrils were adjusted with the buffer to a concentration of 25 μM and then ultrasonicated for 10 minutes, whereby an amyloid β fibril-containing solution was obtained.

[Method of Measuring Amyloid β Fibril Decomposition]

The above-prepared amyloid β fibril containing solution (179 parts by volume) and dry earthworm powder extract (1 part by volume) were mixed and incubated at 37°C. This mixed solution was sampled after 0 and 240 minutes respectively. To 180 μL of each sampled mixed solution, 30 μL of 200 μM thioflavin T (hereinafter, referred to as "ThT"; manufactured by Sigma-Aldrich Co. LLC., T3516-5G, Lot. 088K1665) was added, and the amount of fluorescence emitted by ThT through binding with the amyloid β fibrils was measured three times using a fluorophotometer (VersaFluor, manufactured by Bio-Rad Laboratories, Inc.; using a 440-nm excitation wavelength filter and a 485-nm measurement wavelength filter). From the average value of the thus measured amount of fluorescence emitted by ThT, the fluorescence emission ratio after 240 minutes was calculated using the following equation, taking the fluorescence emission ratio at 0 hours as 100%. It is noted here that ThT is an organic small molecule that emits fluorescence by specifically binding to an amyloid fibril.

$$\text{Fluorescence emission ratio after 240 minutes (\%)} = \frac{\text{Amount of emitted fluorescence after 240 minutes (average)}}{\text{Amount of emitted fluorescence at 0 minutes (average)}} \times 100$$

In the same manner, as a control, the above-prepared amyloid β fibril containing solution was mixed with the buffer, not with the dry earthworm powder extract, and the amount of fluorescence emitted by ThT was measured and the fluorescence emission ratio after 240 minutes was calculated.

Tables 1 and 2 show the amount of fluorescence emitted by ThT for each sample. In addition, Table 3 and FIG. 1 show the fluorescence emission ratio after 240 minutes.

[Table 1]

Amount of fluorescence emitted by ThT in amyloid β fibril containing solutions

| | 0 minutes | 240 minutes |
|---|---|---|
| Comparative Example 1 | 2,364 | 1,894 |
| Comparative Example 2 | 2,363 | 1,829 |
| Comparative Example 3 | 2,381 | 1,856 |
| Average | 2,369 | 1,860 |

[Table 2]

Amount of fluorescence emitted by ThT in mixed solutions of amyloid β fibril containing solution and dry earthworm powder extract

| | 0 minutes | 240 minutes |
|---|---|---|
| Example 1 | 2,347 | 1,035 |
| Example 2 | 2,391 | 962 |
| Example 3 | 2,397 | 986 |
| Average | 2,378 | 994 |

[Table 3]

Comparison of ThT fluorescence emission ratios after 240 minutes

| | 0 minutes | 240 minutes |
|---|---|---|
| Amyloid β fibril containing solution | 100.00% | 78.49% |
| Mixed solution of amyloid β fibril containing solution and dry earthworm powder extract | 100.00% | 41.81% |

Since the ThT fluorescence emission ratio was largely reduced in the mixed solutions containing the dry earthworm powder extract, it is understood that the dry earthworm powder exerts an action of decomposing the amyloid β fibrils.

## Claims

1. An amyloid β fibril decomposing agent comprising a dry powder, ground product and/or extract of an earthworm as an active ingredient.

2. A therapeutic or preventive agent for a disease caused by amyloid β fibril formation, said agent comprising the amyloid β fibril decomposing agent according to claim 1.

3. A therapeutic or preventive food composition for a disease caused by amyloid β fibril formation, said food composition comprising the amyloid β fibril decomposing agent according to claim 1.

4. The therapeutic or preventive agent according to claim 2, wherein said disease caused by amyloid β fibril formation is Alzheimer's disease.

5. The therapeutic or preventive food composition according to claim 3, wherein said disease caused by amyloid β fibril formation is Alzheimer's disease.

6. A method of producing an amyloid β fibril decomposing agent, said method comprising the use of a dry powder, ground product and/or extract of an earthworm.

7. The method of producing an amyloid β fibril decomposing agent according to claim 6, said method comprising the steps of:

   bringing a live earthworm into contact with a chloride of at least one metal selected from the group consisting of potassium, sodium, magnesium and calcium; and
   subsequently bringing said live earthworm into contact with a powder-form hydroxycarboxylic acid, diluting the resultant with water to adjust the pH to 2 to 5, maintaining the resulting dilution for 3 to 180 minutes, washing said live earthworm with water, grinding said live earthworm, freeze-drying the resulting ground product, dissolving the thus freeze-dried product in water or an aqueous ethanol solution, and then removing or separating insoluble fractions.

8. The method of producing an amyloid β fibril decomposing agent according to claim 6, said method comprising the steps of:

bringing a live earthworm into contact with a chloride of at least one metal selected from the group consisting of potassium, sodium, magnesium and calcium; and
subsequently immersing and maintaining said live earthworm for 3 to 180 minutes in an aqueous hydroxycarboxylic acid solution having an adjusted pH of 2 to 5, washing said live earthworm with water, grinding said live earthworm, freeze-drying the resulting ground product, dissolving the thus freeze-dried product in water or an aqueous ethanol solution, and then removing or separating insoluble fractions.

**9.** A method of producing a therapeutic or preventive agent for a disease caused by amyloid $\beta$ fibril formation, said method comprising the use of a dry powder, ground product and/or extract of an earthworm.

**10.** A method of producing a therapeutic or preventive food composition for a disease caused by amyloid $\beta$ fibril formation, said method comprising the use of a dry powder, ground product and/or extract of an earthworm.

**11.** A method of decomposing amyloid $\beta$ fibrils, said method comprising the use of a dry powder, ground product and/or extract of an earthworm.

**12.** A dry powder, ground product or extract of an earthworm for use in the treatment of a disease caused by amyloid $\beta$ fibril formation.

**13.** A method of treating or preventing a disease caused by amyloid $\beta$ fibril formation, said method comprising administration of a dry powder, ground product and/or extract of an earthworm to a subject in an effective dose.

FIG. 1

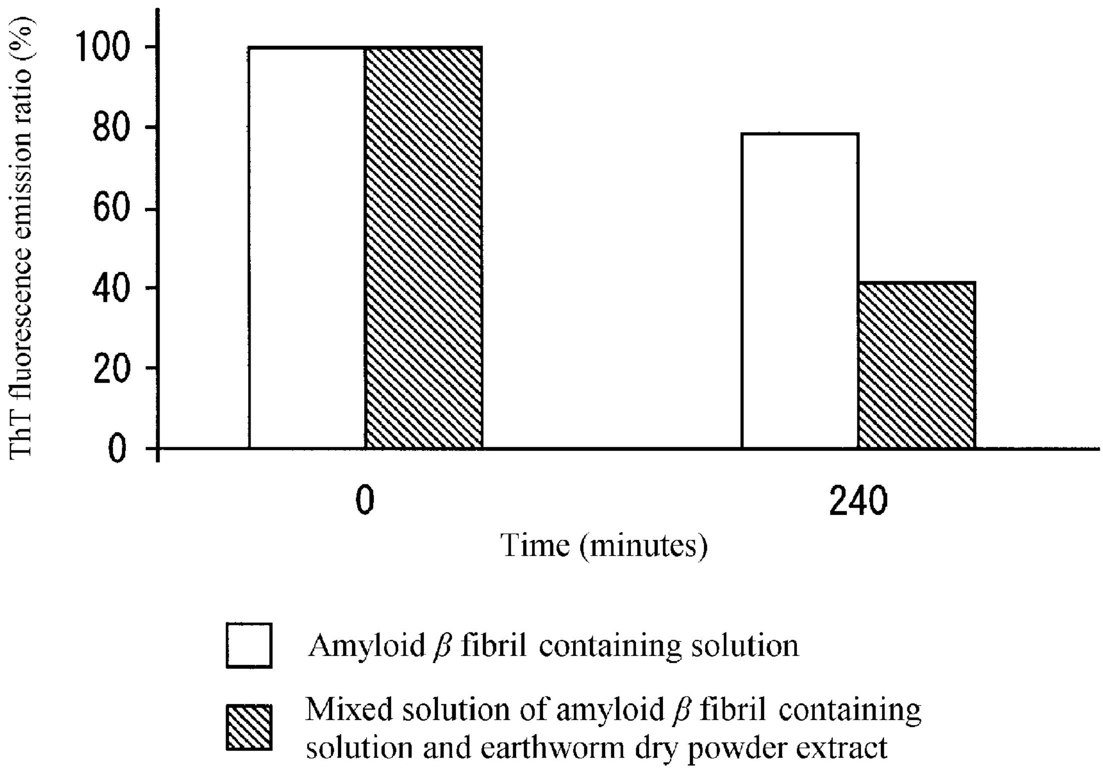
ThT fluorescence emission ratio (%)
100
80
60
40
20
0
0
240
Time (minutes)
Amyloid β fibril containing solution
Mixed solution of amyloid β fibril containing solution and earthworm dry powder extract

## INTERNATIONAL SEARCH REPORT

International application No.
PCT/JP2016/070902

A. CLASSIFICATION OF SUBJECT MATTER
*A61K35/62*(2006.01)i, *A23L33/10*(2016.01)i, *A61P3/00*(2006.01)i, *A61P25/28*(2006.01)i

According to International Patent Classification (IPC) or to both national classification and IPC

B. FIELDS SEARCHED

Minimum documentation searched (classification system followed by classification symbols)
A61K35/62, A23L33/10, A61P3/00, A61P25/28

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII), CAplus/MEDLINE/EMBASE/BIOSIS/WPIDS(STN), CiNii, Japio-GPG/FX

C. DOCUMENTS CONSIDERED TO BE RELEVANT

| Category* | Citation of document, with indication, where appropriate, of the relevant passages | Relevant to claim No. |
|---|---|---|
| X/Y | NAKAJIMA, N., et al., Further Characterization of Earthworm Serine Proteases: Cleavage Specificity Against Peptide Substrates and on Autolysis, Biosci. Biotechnol. Biochem., 1999, Vol.63, No.11, pp.2031-2033 | 1-6,9-13/ 1-13 |
| X/Y | CN 104547388 A (HEILONGJIANG RUTAI BIOLOGICAL PHARMACEUTICAL CO., LTD.), 29 April 2015 (29.04.2015), paragraphs [0111] to [0167] (Family: none) | 1-6,9,10,12, 13/1-13 |
| X/Y | CN 104288396 A (SUI RONGLING), 21 January 2015 (21.01.2015), paragraph [0002] (Family: none) | 1-6,9,10,12, 13/1-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 August 2016 (12.08.16) | 30 August 2016 (30.08.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No. PCT/JP2016/070902

C (Continuation). DOCUMENTS CONSIDERED TO BE RELEVANT

| Category* | Citation of document, with indication, where appropriate, of the relevant passages | Relevant to claim No. |
|---|---|---|
| X/Y | CN 103550537 A (MENG XIAOBIN), 05 February 2014 (05.02.2014), abstract; paragraph [0002] (Family: none) | 1-6,9,10,12, 13/1-13 |
| X/Y/A | WO 2013/018587 A1 (Well Stone Co.), 07 February 2013 (07.02.2013), claims & US 2014/0154331 A1 claims | 12/7,8/1-6, 9-11,13 |
| P,X/P,Y | WO 2015/108014 A1 (Well Stone Co.), 23 July 2015 (23.07.2015), claims; examples & AU 2015207191 A & CA 2935315 A | 1-13/1-13 |
| P,X/P,Y | WO 2016/072392 A1 (Well Stone Co.), 12 May 2016 (12.05.2016), claims; paragraphs [0033] to [0066] & JP 2016-88877 A | 1-13/1-13 |
| A | JP 2008-081476 A (Well Stone Co.), 10 April 2008 (10.04.2008), & TW 200940076 A | 1-13 |
| A | JP 2009-249362 A (Well Stone Co.), 29 October 2009 (29.10.2009), (Family: none) | 1-13 |
| A | JP 2011-084483 A (Well Stone Co.), 28 April 2011 (28.04.2011), & US 2011/0086106 A1 | 1-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4227275 B
- JP H8283157 B
- JP 5464383 B
- JP H147718 B
- JP H147719 B
- JP H147720 B
- JP H1268639 B
- JP H372427 B


### Non-patent literature cited in the description


- **ASAMI-ODAKA, A et al.** *Biochemistry,* 1995, vol. 34 (32), 10272-10278
- **IWATSUBO, T et al.** *Neuron,* 1994, vol. 13 (1), 45-53